# EUROPEAN PATENT APPLICATION

(11) **EP 4 570 790 A1**
(43) Date of publication of application: **18.06.2025**
(21) Application number: 23873066.7
(22) Date of filing: 26.09.2023
(51) Int. Cl.: C07D 307/48

(54) **5-HALOMETHYLFURFURAL PRODUCTION METHOD INVOLVING PRETREATMENT STEP**

(30) Priority: 28.09.2022 KR 20220123130
(71) Applicant: Korea Research Institute of Chemical Technology, Daejeon 34114 (KR)
(72) Inventor: HWANG, Dong Won, Daejeon 34114 (KR); EOM, In Yong, Daejeon 34114 (KR); LEE, Ma Eum, Daejeon 34114 (KR); KIM, Ji Hoon, Daejeon 34114 (KR); YOO, Chang Ho, Daejeon 34114 (KR); HWANG, Young Kyu, Daejeon 34114 (KR); YUN, Gwang Nam, Daejeon 34114 (KR); CHA, Seung Hyeok, Daejeon 34114 (KR)
(74) Representative: Isarpatent
(86) International application number: PCT/KR2023/014749
(87) International publication number: WO 2024/071951

(57) **Abstract**

The present invention relates to a 5-halomethylfurfural production method involving a pretreatment step and, specifically, to a 5-halomethylfurfural production method whereby the yield of halomethylfurfural can be maximized by performing a pretreatment that improves the degree of solubilization of a sugar component before dehydration.

## Description

### Technical Field

The present disclosure relates to a method for producing 5-halomethylfurfural involving a pretreatment step. More specifically, the present disclosure relates to a method for producing 5-halomethylfurfural, the method being capable of maximizing a yield of 5-halomethylfurfural by performing a pretreatment method, which is to improve solubility of a sugar component before a dehydration reaction.

### Background Art

An increase in the use of fossil fuels to meet the rapidly increasing demand for energy and chemical raw materials is pointed out as a main cause of global warming and abnormal climate phenomena. Accordingly, techniques using sustainable resources that can replace fossil fuels have been researched and developed.

As an example of a sustainable resource being presented, research is underway to prepare fuels and chemical raw materials using biomass. Biomass contains cellulose, hemicellulose, lignin, and starch abundant in the biological world. In particular, techniques to utilize biomass from non-food crops with the potential to be supplied abundantly and stably have been researched and developed.

5-Hydroxymethylfurfural (hereinafter referred to as HMF) is one of the high value-added chemical raw materials derived from waste wood. 5-Hydroxymethylfurfural does not have many uses in itself. However, 2,5-furandicarboxylic acid, which is a main HMF derivative, can replace terephthalic acid, which is a polyester raw material. 5-dimethylfuran as another derivative is a high-value-added chemical raw material with a potential to be used as a biofuel because 5-dimethylfuran has a higher energy content than bioethanol.

The HMF is an intermediate produced in the process of producing a levulinic acid. Herein, the levulinic acid is produced through a dehydration reaction of a component containing a 6-carbon sugar in the presence of an acid catalyst. However, when hemicellulose containing xylose is used as a raw material, it is required to perform a biological conversion process using microorganisms, and there is difficulty in commercialization due to a low rate, yield, and stability of the biological conversion process.

Accordingly, US Patent No. 7829732 (registered on November 9, 2010) discloses a technique to dehydrate biomass containing cellulose and hemicellulose to obtain a product in the form of 5-chloromethylfurfural (CMF). In detail, the technique disclosed is about a method of creating a two-phase system of an aqueous phase and an organic phase by injecting an organic solvent, which is an extraction solvent, into the reaction system, and then generating 5-chloromethylfurfural (CMF) from biomass using an acid catalyst and chlorine ions in the aqueous phase, and recovering the produced 5-chloromethylfurfural (CMF) by transferring it from the aqueous phase to the organic phase. However, in the case of hydrochloric acid (HCl) used as an acid catalyst described in Examples in the conventional literature, high-pressure vessel (autoclave) equipment is not only required, but safety accidents such as explosions or hydrochloric acid gas leaks may occur during the production process of 5-chloromethylfurfural (CMF). Above all, polysaccharides are not easy to solubilize in a reaction system containing HCl. Because of that, the dehydration reaction efficiency is low, and a yield of CMF deteriorates accordingly.

Regarding the CMF production method using sulfuric acid instead of HCl as an acid catalyst, Chinese Registered Patent No. 102391218 (published on December 25, 2013) and Chinese Patent Application Publication No. 112321547 (published on February 5, 2021) disclose a method of producing CMF by simultaneously adding biomass raw materials and acid catalysts such as sulfuric acid and Cl salt into a reactor and then raising the temperature. However, there is a limitation to increasing the yield of halomethylfurfural produced through dehydration reaction due to low solubility of polysaccharide components contained in biomass in sulfuric acid. Accordingly, there is a need for a method for producing halomethylfurfural capable of being commercialized with an excellent yield through a simple pretreatment process.

### Disclosure

### Technical Problem

The objective of the present disclosure is to provide a method for producing 5-halomethylfurfural, the method capable of maximizing a yield of halomethylfurfural by performing a pretreatment method, which is to improve solubility of a sugar component before a dehydration reaction.

### Technical Solution

To solve the problem, a method for producing 5-halomethylfurfural involving a pretreatment step of the present disclosure may include: (a) pre-treating a sugar aqueous solution by stirring a mixture of a sugar component and a sulfuric acid aqueous solution for a predetermined time; (b) inducing 5-halomethylfurfural production by adding a halogen-containing inorganic salt and an extraction organic solvent to the sugar aqueous solution, and then obtaining a two-phase solution, and increasing temperature of the two-phase solution; and (c) bringing the two-phase solution to stand after the step (b), dividing the two phases of the solution into an aqueous phase and an organic phase, and recovering 5-halomethylfurfural from the organic phase.

The sugar component in the step (a) may be a raw material component that may be converted to halomethylfurfural or HMF. The sugar component may include one or more selected from the group consisting of monosaccharides such as glucose, fructose, and galactose; disaccharides such as maltose, sucrose, and lactose; and polysaccharides such as starch containing hexoses, cellulose, and hemicellulose.

The sugar component may be derived from biomass. The biomass for use may include one or more selected from the group consisting of agricultural products, such as fruits, peels, stems, and mixtures thereof, or biological raw materials grown naturally or artificially; and processed products, food, or waste derived from the agricultural products or biological raw materials.

The sulfuric acid in the step (a) may have a concentration of 40 to 80 wt% based on the total weight of the sulfuric acid aqueous solution.

The halogen-containing inorganic salt in the step (a) may include one or more selected from the group consisting of alkali metal chlorides such as NaCl, LiCl, KCl, RbCl, and CsCl; alkaline earth metal chlorides such as MgCl₂, CaCl₂, SrCl₂, and BaCl₂; alkali metal bromides such as NaBr, LiBr, KBr, RbBr, and CsBr; alkaline earth metal bromides such as MgBr₂, CaBr₂, SrBr₂, and BaBr₂; alkali metal iodides such as NaI, LiI, KI, RbI, and CsI; alkaline earth metal iodides such as MgI₂, CaI₂, SrI₂, and BaI₂; alkali metal fluorides such as NaF, LiF, KF, RbF, and CsF; and alkaline earth metal fluorides such as MgF₂, CaF₂, SrF₂, and BaF₂.

The extraction organic solvent in the step (b) may include one or more selected from the group consisting of substituted or unsubstituted monocyclic aromatic compounds having 6 to 20 carbon atoms, such as toluene, benzene, xylene, and mesitylene; substituted or unsubstituted polycyclic aromatic compounds having 10 to 20 carbon atoms, such as phenanthrene, naphthalene, anthracene, and chrysene; and substituted or unsubstituted aliphatic compounds having 5 to 30 carbon atoms, such as pentane, hexane, and heptane; substituted or unsubstituted alcohol compounds having 1 to 20 carbon atoms; substituted or unsubstituted ether compounds having 2 to 40 carbon atoms; and substituted or unsubstituted ester compounds having 2 to 40 carbon atoms.

The two-phase solution of the step (b) may have a volume ratio of the organic phase/aqueous phase of 1 to 10.

The two-phase solution may have a temperature raised to 80°C to 160°C in the step (b).

In another embodiment of the present disclosure, the extraction organic solvent added in the step (b) may be previously added to the pretreatment step of the step (a) for the mixture of the sugar component and sulfuric acid aqueous solution, and only the halogen-containing inorganic salt may be further added in the step (b).

As a method for producing 5-hydroxymethylfurfural (HMF) or 5-acetoxymethylfurfural (AMF) from a sugar component, the present disclosure may provide a method for producing 5-hydroxymethylfurfural (HMF) or 5-acetoxymethylfurfural (AMF) using 5-halomethylfurfural produced by the production method of the present disclosure.

### Advantageous Effects

According to the present disclosure, a yield of halomethylfurfural can be improved by simply performing a simple pretreatment process of stirring a mixture for a predetermined time after adding a sugar component and sulfuric acid, which is an acid catalyst, in a reactor. The present disclosure shows a significant effect of improving the halomethylfurfural yield by up to 10% or more compared to the comparative production methods with no performance of a stirring process.

In addition, the present disclosure can establish a commercial production process for 5-halomethylfurfural using a sugar component abundant in biomass. The halomethylfurfural preparation process can be performed by utilizing eco-friendly raw materials derived from biomass with no limitations on abundance, and also be easily applied to the production process of 5-hydroxymethylfurfural (HMF), which is a high-value-added chemical raw material.

### Description of Drawings

Figure 1 shows a graph showing CMF yields during a dehydration reaction with or without a pretreatment step of stirring a cellulose and acid catalyst; and
Figure 2 shows a graph showing BMF yields during a dehydration reaction with or without a pretreatment step of stirring a cellulose and acid catalyst.

### Best Mode

All technical and scientific terms used in this specification have the same meaning as commonly understood by those skilled in the art to which the present disclosure pertains. In general, the nomenclature used herein is well known and commonly used in the art.

Throughout the specification herein, when a part is said to "include" a certain component, this means that it may further include other components rather than excluding other components, unless specifically stated to the contrary.

Hereinafter, the method for producing 5-halomethylfurfural involving a pretreatment step of the present disclosure will be examined.

The method for producing 5-halomethylfurfural involving a pretreatment step of the present disclosure includes: (a) pre-treating a sugar aqueous solution by stirring a mixture of a sugar component and a sulfuric acid aqueous solution for a predetermined time; (b) inducing 5-halomethylfurfural production by adding a halogen-containing inorganic salt and an extraction organic solvent to the sugar aqueous solution, and then obtaining a two-phase solution, and increasing temperature of the two-phase solution; and (c) bringing the two-phase solution to stand after the step (b), dividing the two phases of the solution into an aqueous phase and an organic phase, and recovering 5-halomethylfurfural from the organic phase.

The step (a) involves pre-treating a sugar aqueous solution by stirring the mixture of the sugar component and the sulfuric acid aqueous solution for a predetermined time.

The sugar component is a raw material component that may be converted to halomethylfurfural or HMF. The sugar component includes one or more selected from the group consisting of monosaccharides such as glucose, fructose, and galactose; disaccharides such as maltose, sucrose, and lactose; and polysaccharides such as starch containing hexoses, cellulose, and hemicellulose.

In addition, it is preferable to use a sugar component derived from biomass in terms of environmentally friendly raw material supply. The biomass for use may include one or more selected from the group consisting of agricultural products, such as fruits, peels, stems and mixtures thereof, or biological raw materials grown naturally or artificially; and processed products, food or waste derived from the agricultural products or biological raw materials. However, sugar components supplied from other sources may also be used as the biomass without limitation.

The stirring in the step (a) is involved in the pretreatment step in which the sugar component is added to the sulfuric acid aqueous solution, which is an acid catalyst, and then the mixture is stirred. The stirring time is at least 1 minute, preferably at least 10 minutes. The pretreatment temperature for stirring is 5°C to 50°C, preferably 15°C to 30°C including room temperature.

The sulfuric acid aqueous solution in the step (a) is non-volatile with almost no sulfuric acid present in a gas phase until the concentration of H₂SO₄ reaches 70 wt%, and even in the case of 98 wt% concentrated sulfuric acid, the H₂SO₄ concentration in a gas phase remains lower than the concentration in a liquid phase. Thus, the production reaction of 5-halomethylfurfural from a sugar component may be easily performed without the need for an autoclave equipment. By providing a simple solvent reflux system, 5-halomethylfurfural may be produced easily and in high yield with a general apparatus without a special temperature control system.

The concentration of sulfuric acid in the step (a) may be in the range of 0.1 to 98 wt% based on the total weight of the aqueous sulfuric acid solution, and preferably in the range of 40 to 80 wt%.

The halogen-containing inorganic salt in the step (a) is used as a halogen source to replace HMF. The halogen-containing inorganic salt is a component introduced by replacing hydrochloric acid, a conventional acid catalyst, with sulfuric acid. The inorganic salt, which is an ionic substance, is not only used as a halogen reaction raw material for the production of 5-halomethylfurfural, but is also a component that increases hydrophilicity of the aqueous phase in the two-phase solution, promoting the distribution of 5-halomethylfurfural into the organic phase, and as a result, improving yield. The halogen-containing inorganic salt may contain a metal ion, and the metal ion forms a salt with sulfate ion (SO₄²⁻), causing a salting-out effect. Thereby, the halogen-containing inorganic salt has the effect of further promoting the distribution of 5-halomethylfurfural.

The halogen-containing inorganic salt may be used without limitation as long as it may be dissolved to provide halogen ions (X⁻=F⁻, Cl⁻, Br⁻, I⁻). For example, the halogen-containing inorganic salt may be one containing a metal cation and a chlorine anion. More specifically, the halogen-containing inorganic salt may include one or more selected from the group consisting of alkali metal chlorides such as NaCl, LiCl, KCl, RbCl, and CsCl; alkaline earth metal chlorides such as MgCl₂, CaCl₂, SrCl₂, and BaCl₂; alkali metal bromides such as NaBr, LiBr, KBr, RbBr, and CsBr; alkaline earth metal bromides such as MgBr₂, CaBr₂, SrBr₂, and BaBr₂; alkali metal iodides such as NaI, LiI, KI, RbI, and CsI; alkaline earth metal iodides such as MgI₂, CaI₂, SrI₂, and BaI₂; alkali metal fluorides such as NaF, LiF, KF, RbF, and CsF; and alkaline earth metal fluorides such as MgF₂, CaF₂, SrF₂, and BaF₂. The halogen-containing inorganic salt is preferably one or more selected from the group consisting of alkali metal chlorides and alkaline earth metal chlorides, and even more preferably NaCl.

The step (b) involves inducing a dehydration reaction by adding a halogen-containing inorganic salt and extraction organic solvent to the sugar solution in the step (a), and then obtaining a two-phase solution, and raising the temperature of the two-phase solution. At this time, the pressure in the step (b) may be a normal pressure or a vapor pressure of the extraction organic solvent at each temperature. When the reaction temperature is below a boiling point of a solvent and at the normal pressure, a reaction may be performed while refluxing the solvent.

The extraction organic solvent in the step (b) may include one or more selected from the group consisting of substituted or unsubstituted monocyclic aromatic compounds having 6 to 20 carbon atoms, such as toluene, benzene, xylene, and mesitylene; substituted or unsubstituted polycyclic aromatic compounds having 10 to 20 carbon atoms, such as phenanthrene, naphthalene, anthracene, and chrysene; and substituted or unsubstituted aliphatic compounds having 5 to 30 carbon atoms, such as pentane, hexane, and heptane; substituted or unsubstituted alcohol compounds having 1 to 20 carbon atoms; substituted or unsubstituted ether compounds having 2 to 40 carbon atoms; and substituted or unsubstituted ester compounds having 2 to 40 carbon atoms, and may preferably be toluene.

As an example of the present disclosure, the extraction organic solvent may be added at the same time with the halogen-containing inorganic salt into the sugar aqueous solution in the step (b). As another example, it is possible to add the extraction organic solvent in advance in the pretreatment step of the step (a) for the mixture of the sugar component and sulfuric acid aqueous solution.

The two-phase solution of the step (b) may have a volume ratio of the organic phase/aqueous phase of 1 to 10.

In the step (b), the two-phase solution may have a temperature raised to 80°C to 160°C. When the raised temperature is less than 80°C, there is a problem that the dehydration reaction rate may be insignificant. When the raised temperature is higher than 160°C, not only may a high-temperature and high-pressure reaction facility be required, but there is a problem that over-decomposition reaction may be promoted.

In the step (b), stirring may be performed on the two-phase solution to expand the interface where the dehydration reaction occurs from the viewpoint of improving the reaction rate and yield.

The step (c) involves bringing the two-phase solution to stand after the step (b), dividing the two phases of the solution into an aqueous phase and an organic phase, and recovering 5-halomethylfurfural from the organic phase. At this time, it is preferable to rapidly cool the reaction mixture after the reaction in the step (b) in terms of suppressing reaction by-products. The rapid cooling method is not limited as long as it may rapidly cool the reaction mixture. For example, the rapid cooling method may be a method of directly immersing a reaction mixture container in a cold water bath at 0°C or a method of cooling a reaction mixture by discharging the reaction mixture into a separate container immersed in a cold water bath at 0°C. The method of recovering 5-halomethylfurfural from the organic phase is not particularly limited, and for example, a method such as chromatography may be applied.

Additionally, the present disclosure provides a method for producing 5-hydroxymethylfurfural (HMF) in high yield from a sugar component. The method for producing 5-hydroxymethylfurfural (HMF) of the present disclosure is to obtain 5-hydroxymethylfurfural (HMF) using 5-halomethylfurfural produced according to the method for producing 5-halomethylfurfural of the present disclosure described above.

Additionally, the present disclosure provides a method for producing 5-acetoxymethylfurfural (AMF) in high yield from a sugar component. The method for producing 5-acetoxymethylfurfural (AMF) of the present disclosure is to obtain 5-acetoxymethylfurfural (AMF) using 5-halomethylfurfural produced according to the method for producing 5-halomethylfurfural of the present disclosure described above.

The 5-halomethylfurfural prepared according to the present disclosure may be easily converted to the 5-hydroxymethylfurfural (HMF) or 5-acetoxymethylfurfural (AMF) by a known method while suppressing the production of other by-products. Therefore, when the 5-halomethylfurfural may be produced from a sugar component by the method for obtaining 5-halomethylfurfural of the present disclosure, and the 5-halomethylfurfural produced above is converted back to 5-hydroxymethylfurfural (HMF) or 5-acetoxymethylfurfural (AMF), it is possible to avoid the production of excessive by-products during the direct production of 5-hydroxymethylfurfural (HMF) or 5-acetoxymethylfurfural (AMF) from a sugar component. Thus, it is possible to produce 5-hydroxymethylfurfural (HMF) or 5-acetoxymethylfurfural (AMF) in high yield.

Hereinafter, preferred examples of the method for producing 5-halomethylfurfural involving the pretreatment step of the present disclosure will be examined. For reference, the following examples are provided to illustrate one or more preferred examples of the present disclosure but are not intended to limit the present disclosure to those examples. Numerous modifications may be made to the following examples within the scope of the present disclosure.

### <Example 1>

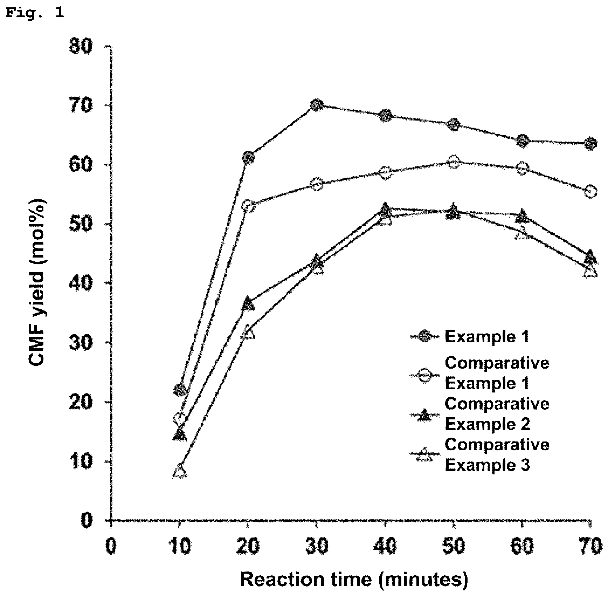

A mixture of 0.12 g cellulose and 1 mL sulfuric acid aqueous solution with a concentration of 67 wt% in a pressure glass vessel (Chemglass) was stirred at room temperature for 4 hours, and an aqueous cellulose solution was obtained. Thereafter, 0.45 g NaCl and 6 mL toluene were further added thereto to prepare a two-phase solution. The two-phase solution was heated to 110°C while stirring to perform a dehydration reaction. After a predetermined reaction time had passed, the reaction flask was placed in a cold water bath at 0°C and rapidly cooled, and then a toluene layer was separated from a water layer. Anhydrous magnesium sulfate was added to the toluene layer to remove moisture, and then, the mixture was filtered to obtain toluene. The obtained toluene was distilled under reduced pressure (room temperature, <50 mmHg), and a non-volatile 5-chloromethylfurfural (CMF) stock solution was recovered. Column chromatography (silica gel, CH₂Cl₂:Et₂O, 2:1) was performed on the recovered 5-chloromethylfurfural (CMF) stock solution. Through this, high-purity 5-chloromethylfurfural (CMF) was finally recovered. For quantitative analysis of the 5-chloromethylfurfural (CMF) produced in the toluene layer, using a response factor measured between isolated high-purity 5-chloromethylfurfural (CMF) and 1-heptane, which serves as an internal standard, the 5-chloromethylfurfural (CMF) produced in the toluene layer was quantified through GC analysis equipped with a DB-624UI column and FID. The yield of the 5-chloromethylfurfural (CMF) is shown in Figure 1.

### <Comparative Example 1>

5-chloromethylfurfural (CMF) was produced in the same manner as Example 1, except that equal amounts of NaCl and toluene were added immediately without stirring after mixing cellulose and sulfuric acid aqueous solution.

### <Comparative Example 2>

5-Chloromethylfurfural (CMF) was produced in the same manner as Example 1, except that a hydrochloric acid aqueous solution was used instead of a sulfuric acid aqueous solution, and NaCl was not used.

### <Comparative Example 3>

5-chloromethylfurfural (CMF) was produced in the same manner as Comparative Example 2, except that toluene was immediately added without stirring the mixture of cellulose and hydrochloric acid aqueous solution.

Figure 1 is a graph showing CMF yields during a dehydration reaction with or without a pretreatment step of stirring a cellulose and acid catalyst. Referring to FIG. 1, in Example 1 with the addition of sulfuric acid an acid catalyst and stirring pretreatment, the CMF yield was found to be improved under a reaction time of 10 minutes or more compared to Comparative Example 1 without stirring pretreatment. In particular, under conditions in which the reaction time was 20 minutes or more, the CMF yield of Example 1 was 60% or more, and the yield of Example 1 was improved by up to 13% or more compared to Comparative Example 1.

On the other hand, Comparative Example 2 with the addition of HCl as an acid catalyst and stirring pretreatment showed the effect of improving a CMF yield under the reaction time of 10 minutes or more compared to Comparative Example 3 without stirring pretreatment. However, as the reaction time passed, it was confirmed that the difference in the CMF yield became significantly slight.

Therefore, when sulfuric acid was used as the acid solution as in the present disclosure, the CMF yield could be improved by pretreatment in advance.

### <Example 2>

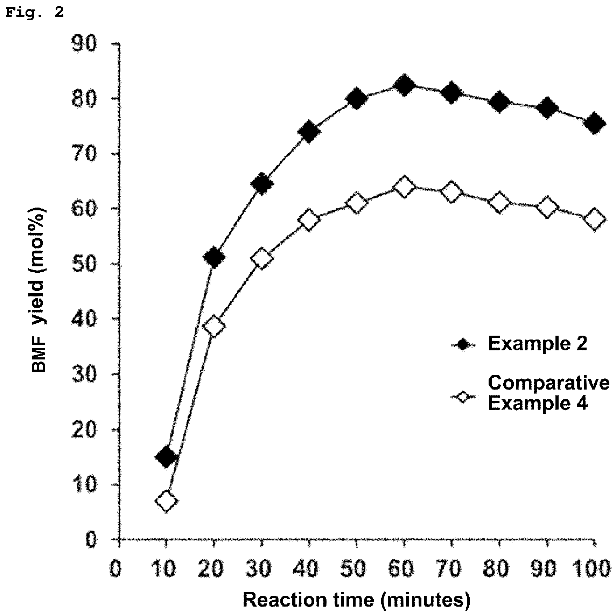

A mixture of 12.0 g cellulose and 125 mL sulfuric acid aqueous solution with a concentration of 67 wt% was stirred for 1 hour at room temperature in a 2-liter three-necked flask equipped with a condenser, and an aqueous cellulose solution was obtained. Thereafter, 22.2 g NaBr and 1000 mL toluene were further added thereto to prepare a two-phase solution. The two-phase solution was heated to 110°C, which is the boiling point of toluene, while stirring to perform a dehydration reaction. After a predetermined reaction time had passed, the reaction flask was placed in a cold water bath at 0°C and rapidly cooled, and then a toluene layer was separated from a water layer. Anhydrous magnesium sulfate was added to the toluene layer to remove moisture, and then the mixture was filtered to obtain toluene. The obtained toluene was distilled under reduced pressure (room temperature, <50 mmHg), and a non-volatile 5-bromomethylfurfural (BMF) stock solution was recovered. Column chromatography (silica gel, CH₂Cl₂:Et₂O, 2:1) was performed on the recovered 5-bromomethylfurfural (BMF) stock solution. Through this, high-purity 5-bromomethylfurfural (BMF) was finally recovered. For quantitative analysis of the 5-bromomethylfurfural (BMF) produced in the toluene layer, using a response factor measured between isolated high-purity 5-bromomethylfurfural (BMF) and 1-heptane, which served as an internal standard, the 5-bromomethylfurfural (BMF) produced in the toluene layer was quantified through GC analysis equipped with a DB-624UI column and FID. The yield of the 5-bromomethylfurfural (BMF) is shown in Figure 2.

### <Comparative Example 4>

5-bromomethylfurfural (BMF) was produced in the same manner as Example 2, except that equal amounts of NaBr and toluene were added immediately without stirring after mixing cellulose and sulfuric acid aqueous solution. The yield is shown in Figure 2.

Referring to Figure 2, even in the case of producing 5-bromomethylfurfural (BMF) from cellulose using Br as a halogen element, in Example 2 with the addition of sulfuric acid as an acid catalyst and stirring pretreatment, a BMF yield was improved throughout a reaction time compared to Comparative Example 4 without stirring pretreatment. It was confirmed that the highest yield of BMF in Example 2 was also significantly improved compared to the case without the pretreatment.

As above, the present disclosure has been described with reference to the examples described in the specification or shown in the attached drawings, but this is merely illustrative. Those skilled in the art will understand that various modifications and other equivalent examples are possible. Therefore, the scope of technical protection of the present disclosure should be determined by the scope of the patent claims below.

## Claims

1. A method for producing 5-halomethylfurfural comprising a pretreatment step, the method comprising:
(a) pre-treating a sugar aqueous solution by stirring a mixture of a sugar component and a sulfuric acid aqueous solution for a predetermined time;
(b) inducing 5-halomethylfurfural production by adding a halogen-containing inorganic salt and an extraction organic solvent to the sugar aqueous solution, and then obtaining a two-phase solution, and increasing temperature of the two-phase solution; and
(c) bringing the two-phase solution to stand after the step (b), dividing the two phases of the solution into an aqueous phase and an organic phase, and recovering 5-halomethylfurfural from the organic phase.

2. The method of Claim 1, wherein the sugar component comprises one or more selected from the group consisting of monosaccharides such as glucose, fructose, and galactose; disaccharides such as maltose, sucrose, and lactose; and polysaccharides such as starch containing hexoses, cellulose, and hemicellulose.

3. The method of Claim 2, wherein the sugar component is derived from biomass,
wherein the biomass for use comprises one or more selected from the group consisting of:
agricultural products, such as fruits, peels, stems, and mixtures thereof, or biological raw materials grown naturally or artificially; and
processed products, food, or waste derived from the agricultural products or biological raw materials.

4. The method of Claim 1, wherein the sulfuric acid in the step (a) has a concentration of 40 to 80 wt% based on the total weight of the sulfuric acid aqueous solution.

5. The method of Claim 1, wherein the halogen-containing inorganic salt in the step (a) comprises one or more selected from the group consisting of:
alkali metal chlorides such as NaCl, LiCl, KCl, RbCl, and CsCl;
alkaline earth metal chlorides such as MgCl₂, CaCl₂, SrCl₂, and BaCl₂;
alkali metal bromides such as NaBr, LiBr, KBr, RbBr, and CsBr; alkaline earth metal bromides such as MgBr₂, CaBr₂, SrBr₂, and BaBr₂;
alkali metal iodides such as NaI, LiI, KI, RbI, and CsI;
alkaline earth metal iodides such as MgI₂, CaI₂, SrI₂, and BaI₂;
alkali metal fluorides such as NaF, LiF, KF, RbF, and CsF; and
alkaline earth metal fluorides such as MgF₂, CaF₂, SrF₂, and BaF₂.

6. The method of Claim 1, wherein the extraction organic solvent in the step (b) comprises one or more selected from the group consisting of:
substituted or unsubstituted monocyclic aromatic compounds having 6 to 20 carbon atoms, such as toluene, benzene, xylene, and mesitylene;
substituted or unsubstituted polycyclic aromatic compounds having 10 to 20 carbon atoms, such as phenanthrene, naphthalene, anthracene, and chrysene; and
substituted or unsubstituted aliphatic compounds having 5 to 30 carbon atoms, such as pentane, hexane, and heptane;
substituted or unsubstituted alcohol compounds having 1 to 20 carbon atoms;
substituted or unsubstituted ether compounds having 2 to 40 carbon atoms; and
substituted or unsubstituted ester compounds having 2 to 40 carbon atoms.

7. The method of Claim 1, wherein the two-phase solution of the step (b) has a volume ratio of the organic phase/aqueous phase of 1 to 10.

8. The method of Claim 1, wherein the two-phase solution has a temperature raised to 80°C to 160°C in the step (b).

9. The method of Claim 1, wherein the extraction organic solvent added in the step (b) is previously added to the pretreatment step of the step (a) for the mixture of the sugar component and sulfuric acid aqueous solution, and
only the halogen-containing inorganic salt is further added in the step (b).

10. A method for producing 5-hydroxymethylfurfural (HMF) from a sugar component, the method comprising:
obtaining 5-hydroxymethylfurfural (HMF) by producing 5-halomethylfurfural by the production method of Claim 1 above and then using the produced 5-halomethylfurfural.

11. A method for producing 5-acetoxymethylfurfural (AMF) from a sugar component, the method comprising:
obtaining 5-acetoxymethylfurfural (AMF) by producing 5-halomethylfurfural by the production method of Claim 1 above and then using the produced 5-halomethylfurfural.
